# EUROPÄISCHE PATENTANMELDUNG

(11) **EP 4 344 672 A1**
(43) Veröffentlichungstag der Anmeldung: **03.04.2024**
(21) Anmeldenummer: 22198302.6
(22) Anmeldetag: 28.09.2022
(51) Int. Cl.: A61F 2/01

(54) **ENDOVASKULÄRE PROTEKTIONSVORRICHTUNG**

(71) Anmelder: Universitätsklinikum Jena, 07747 Jena (DE)
(72) Erfinder: Schulze, Christian, 07743 Jena (DE); Möbius-Winkler, Sven, 04178 Leipzig (DE); Franz, Marcus, 99310 Arnstadt (DE)
(74) Vertreter: Schneiders & Behrendt Bochum

(57) **Zusammenfassung**

Die Erfindung betrifft eine endovaskuläre Protektionsvorrichtung mit einem Protektionselement, umfassend ein selbstexpandierbares Stützelement (2) und ein an dem Stützelement (2) angeordnetes Filterelement (3), sowie mit einem distal an dem Stützelement (2) angeordneten Zuführelement (5), wobei das Protektionselement reversibel von einem expandierten in einen komprimierten Zustand überführbar ist, wobei das Filterelement (3) zumindest im expandierten Zustand an seinem distalen Ende geöffnet und an seinem proximalen Ende geschlossen ist.

## Beschreibung

Die Erfindung betrifft eine endovaskuläre Protektionsvorrichtung in Form eines reversibel implantierbaren Filtersystems, das insbesondere für den Einsatz bei retrograden Interventionen geeignet ist.

Die zunehmende Verfügbarkeit und der breite klinische Einsatz interventioneller, Katheter-basierter Therapieverfahren im endovaskulären und insbesondere im kardiovaskulären Bereich erfordert das stetige Bemühen um die bestmögliche Reduktion assoziierter Komplikationen, vor allem jener mit prognostischer Relevanz.

So besteht bei einer Vielzahl von endovaskulären Eingriffen die Gefahr einer systemischen oder zerebralen Embolisation beispielsweise durch Kalziummaterial oder Thromben, die sich während der Intervention ablösen. Am Beispiel der transvaskulären TAVI-Prozedur, die eine retrograde Sondierung der linken Herzkammer und damit die Passage der Aortenklappe umfasst, führt dies zu Schlaganfallraten im frühen postoperativen Verlauf zwischen 1 und 10%, wobei die Rate an klinisch stummen Ereignissen deutlich höher liegt.

Im Stand der Technik sind verschiedene Protektionssysteme bekannt, die während einer retrograd durchgeführten Intervention im Aortenbogen platziert werden können.

Beispiele hierfür sind das Sentinel, das TriGuard oder das Embrella Device, welche allesamt auf den Schutz vor zerebralen Embolien abzielen, indem sie die hirnversorgenden, aus dem Aortenbogen abgehenden Gefäße adressieren, nicht jedoch andere systemische Embolisationslokalisationen wie beispielsweise die Extremitäten oder Bauchorgane.

Auch wenn die genannten Devices nach aktueller Studienlage sicher in der Anwendung scheinen, so ist der klinische Nutzen beispielsweise hinsichtlich der Reduzierung von Schlaganfälle bislang aber nicht überzeugend.

Devices, die nicht nur die hirnversorgenden Gefäße, sondern die gesamte arterielle Strombahn vor Embolisationen schützen sollen, sind rar und in den Frühphasen der klinischen Entwicklung wie beispielsweise das Embol-X Device oder das Emboliner Device.

Das Embol-X Device wurde ursprünglich für die offene Herzchirurgie mit Einsatz der Herz-Lungen-Maschine entwickelt und steht auf Grund seines Designs sinnvoll allenfalls für transaortale Zugangswege zur Verfügung.

Das Emboliner Device kann transfemoral leicht vorgebracht werden und deckt den gesamten Aortenbogen bis in die Aorta descendens ab und bietet somit sowohl einen systemischen Schutz als auch einen Schutz der Hirngefäße. Erste Studienergebnisse an bislang nur wenigen Patienten sind vielversprechend, wurden bislang aber noch nicht publiziert.

Nachteilig an dem Emboliner Device ist, dass die proximale Öffnung für die Verwendung weiterer Instrumente nur für bestimmte Größen geeignet ist, also limitierend für die Verwendbarkeit beziehunsgweise Auswahl der Instrumente ist.

Problematisch an dem Emboliner Device ist zudem die erhöhte Gefahr der Mobilisation von Kalzium- und Thrombusmaterial im Bereich des Aortenbogens bei der Entfernung des Devices am Ende der Prozedur. Diese erhöhte Gefahr der Mobilisation hängt insbesondere mit der Größe und Komplexität des Devices zusammen, die zu einer großen Kontaktfläche des Filterelements mit dem Gefäß führen und entsprechend bei der Platzierung und Entfernung die Mobilisation von Thrombusmaterial von der Gefäßwand zur Folge haben können.

Insgesamt nachteilig an den bekannten Protektionssystemen ist zusammenfassend, dass sie entweder nur der Hirnprotektion dienen, also keinen Schutz vor systemischen Embolien bieten, oder sie durch ihre Komplexität und ihren Einsatz an sich eine zusätzliche Gefahr der Ablösung von Thromben oder Kalziummaterial darstellen.

Es ist daher Aufgabe der Erfindung, eine Protektionsvorrichtung insbesondere für retrograde endovaskuläre Interventionen zur Verfügung zu stellen, die die bekannten Nachteile nicht aufweist.

Die Erfindung löst diese Aufgabe durch eine endovaskuläre Protektionsvorrichtung mit den Merkmalen des Anspruchs 1. Vorteilhafte Ausgestaltungen sind jeweils Gegenstand der abhängigen Ansprüche. Es ist darauf hinzuweisen, dass die in den Ansprüchen einzeln aufgeführten Merkmale auch in beliebiger und technologisch sinnvoller Weise miteinander kombiniert werden können und somit weitere Ausgestaltungen der Erfindung aufzeigen.

Die erfindungsgemäße Protektionsvorrichtung umfasst ein Protektionselement mit einem selbstexpandierbaren Stützelement und einem an dem Stützelement angeordneten Filterelement. An dem Stützelement ist proximal angeordnet ein Zuführelement vorgesehen.

Das Protektionselement kann reversibel einen komprimierten und einen expandierten Zustand einnehmen und ist entsprechend von einem komprimierten in einen expandierten Zustand überführbar und umgekehrt, wobei das Filterelement zumindest im expandierten Zustand an seinem distalen Ende geöffnet und an seinem proximalen Ende geschlossen ist.

Das Filterelement ist vorzugsweise innerhalb des Stützelements angeordnet und legt sich formschlüssig zumindest in einem bestimmten Bereich an die Innenseite des Stützelements an, vorzugsweise ist dies der proximale Bereich des Stützelements. Stützelement und Filterelement können auf übliche Weisen miteinander verbunden sein, insbesondere kann das Filterelement mit dem Stützelement verklebt, verschweißt, vernäht oder in sonstiger Weise verbunden sein.

Es sind auch Ausführungsformen denkbar, bei denen Stütz- und Filterelement zumindest teilweise miteinander verwoben sind.

Das Stützelement ist selbstexpandierbar vorgesehen und kann beispielsweise Formgedächtnislegierungen oder sonstige Legierungen umfassen, aus denen durch Schneiden, insbesondere durch Laserschneiden, oder Flechten oder sonstige Techniken selbstexpandierende Elemente herstellbar sind.

Unter selbstexpandierenden Elementen sind solche, insbesondere Stent-artige, Elemente zu verstehen, die reversibel einen komprimierten und einen expandierten Zustand annehmen können. Die selbstexpandierbaren Eigenschaften sind nicht zwingend an ein Material mit Formgedächtniseigenschaften gebunden, also beispielsweise an Nickel-Titan-Legierungen wie Nitinol, sondern können auch durch bestimmte Herstellungstechniken bewirkt werden. Entsprechend können auch beispielsweise medizinischer Stahl oder Kobalt-Chrom-Legierungen zur Herstellung des Stützelements geeignet sein.

Das Stützelement weist eine im Wesentlichen hohlzylinderfömige Gestalt auf, wobei sich das Stützelement nach proximal und/oder distal verjüngen und gegebenenfalls jeweils kegelförmig schließen kann. Ähnlich wie ein Stent ist das Stützelement vorzugsweise aus Stegen oder Streben aufgebaut, wobei die Stege oder Streben sich kreuzen und einzelne Zellen beziehungsweise Öffnungen bilden oder im Wesentlichen nebeneinander verlaufen können.

Das Stützelement ist im distalen Bereich grundsätzlich so ausgestaltet, dass thrombembolisches Material in das Filterelement gelangen kann. Das bedeutet, dass auch bei einer sich nach distal verjüngenden oder geschlossenen Bauweise des Stützelements die Zellen beziehungsweise Öffnungen im distalen Bereich des Stützelements ausreichend groß sind, um von thrombembolischen Materialien passiert werden zu können, die sich dann im Filterelement sammeln.

Wesentlich für die Gestaltung des Stützelements ist, dass es eine ausreichende Stützkraft für das Filterelement bietet und sich thrombembolisches Material ungehindert im Filterelement sammeln kann. Ferner soll das implantierte Stützelement in seinem expandierten Zustand der Gefäßwand formschlüssig anliegen.

Am proximalen Ende des Stützelements ist ein Zuführelement vorgesehen, mit dem das Stützelement und entsprechend das Protektionselement insgesamt längs durch einen Zuführkatheter geführt werden kann. Innerhalb des Zuführkatheters nimmt das Protektionselement seinen komprimierten und außerhalb des Zuführkatheters seinen expandierten Zustand ein.

Das Filterelement ist ausreichend flexibel, um der Form des selbstexpandierbaren Stützelements von dem expandierten in den komprimierten Zustand reversibel zu folgen.

Das Filterelement kann aus durchlässigen Materialien mit einer bestimmten Durchlässigkeit beziehungsweise einer bestimmten Porengröße wie Geweben, Membranen oder Fleece oder Kombinationen daraus gefertigt sein, wobei sowohl die Porengröße als auch die letztendliche Materialauswahl den jeweiligen Anforderungen anzupassen ist. Dem Fachmann sind hier verschiedenste geeignete Materialien bekannt, insbesondere verschiedene Polymere wie Polyethylen (PE), Polytetrafluorethylen (PTFE, ePTFE) oder sonstige biokompatiblen Materialien.

In der Regel liegt die Porengröße des Filterelements zwischen 50 und 500 µm, vorzugsweise zwischen 60 und 250 µm und insbesondere zwischen 100 und 150 µm. Das Filterelement kann ein- oder mehrlagig vorgesehen sein.

Sofern das Filterelement mehrlagig vorgesehen ist, können die einzelnen Lagen aus verschiedenen Materialien bestehen beziehungsweise auch verschiedene Materialien umfassen. Die Porengröße der einzelnen Lagen kann unterschiedlich sein.

Das Filterelement kleidet das Stützelement zumindest in dessen proximalem Bereich aus und ist nach proximal geschlossen. Das Filterelement kann das Stützelement auch vollständig von proximal bis nach distal auskleiden. Das Filterelement ist dabei so vorgesehen, dass es in seinem distalen Bereich offen ist und formschlüssig mit dem Stützelement der Gefäßwand anliegt. In seinem proximalen Bereich ist das Filterelement geschlossen, sodass der Blutstrom zwingend das Filterelement durchfließen muss.

Das Protektionselement ist derart gestaltet, dass es bei retrograden Interventionen möglich ist, ein weiteres medizintechnisches Instrument, beispielsweise einen Zuführkatheter, zwischen dem expandierten Protektionselement einerseits und der Gefäßwand, an der das Protektionselement formschlüssig anliegt, andererseits hindurchzuführen.

Weiterhin ist das Protektionselement derart vorgesehen, dass zwischen dem expandierten Protektionselement einerseits und der Gefäßwand andererseits auch dann noch ein weitgehender Formschluss erfolgt, wenn ein weiteres medizintechnisches Instrument, beispielsweise ein Zuführkatheter, zwischen dem expandierten Protektionselement einerseits und der Gefäßwand, an der das Protektionselement formschlüssig anliegt, andererseits anwesend ist.

Um diesen Formschluss jederzeit zu erreichen, sind das Protektionselement beziehungsweise das Stützelement und das Filterelement wie bereits beschrieben aus flexiblen Materialien vorgesehen, die sich an die Form der Gefäßwand und auch an die Form weiterer vorhandener Instrumente anpassen können.

In einer bevorzugten Ausführungsform kann die Protektionsvorrichtung weiterhin eine distal am Protektionselement angeordnete Verschlussvorrichtung umfassen, die vorgesehen ist, einen Verschluss der distalen Öffnung des Stützelements und/oder der distalen Öffnung des Filterelements unabhängig vom sonstigen Expansionszustand des Protektionselements zu ermöglichen.

Dies ist vorteilhaft, damit bereits während des Zurückziehens der Protektionsvorrichtung kein in dem Filterelement eingefangenes Material austreten und gegebenenfalls zu Embolien führen kann.

Die Verschlussvorrichtung kann auf unterschiedliche Weise realisiert sein, beispielsweise in Form eines Tunnelzugs oder einer Tabaksbeutelnaht. Das distale Ende der Protektionsvorrichtung ist entsprechend ausgestaltet.

So umfasst die Verschlussvorrichtung vorzugsweise Halteelemente und zumindest ein Zugelement. Die Halteelemente sind vorzugsweise am distalen Ende des Stützelements oder des Filterelements vorgesehen. Sie können Teil des Stützelements oder des Filterelements sein oder separat an das Stützelement oder das Filterelement angefügt sein.

Selbstverständlich sind auch Ausführungsformen möglich, bei denen die Haltelemente sowohl am Stütz- als auch am Filterelement angeordnet sind.

Die Halteelemente sind zur Aufnahme des Zugelements vorgesehen. Vorzugsweise sind die Haltelemente als Öffnungen oder Ösen vorgesehen, durch die das Zugelement geführt wird, um so die beschriebene Verschlussmöglichkeit beispielsweise in Form eines Tunnelzugs oder einer Tabaksbeutelnaht zu realisieren.

Das Zugelement ist vorzugsweise bandförmig mit zwei Enden vorgesehen. Das Zugelement kann aus jedem geeigneten Material gefertigt sein.

Zumindest ein Ende des Zugelements wird vorzugsweise derart nach proximal durch den Zuführkatheter geführt, dass dieses eine Ende des Zugelements durch den Anwender zum Verschluss des Stütz- und/oder Filterelements genutzt werden kann.

Das andere Ende des Zugelements kann ebenfalls nach proximal durch den Zuführkatheter geführt werden, sodass auch dieses andere Ende des Zugelements durch den Anwender zum Verschluss des Stützelement genutzt werden kann. Das Zugelement bildet dabei entsprechend insgesamt eine Schlinge aus.

Alternativ kann eines der Enden des Zugelements auch an einer Stelle der Protektionsvorrichtung, insbesondere an einer Stelle des Stützelements oder an einem der Halteelemente, festgelegt sein, sodass der Anwender durch Ziehen an dem anderen Ende des Zugelements einen Verschluss des Stütz- und/oder des Filterelements bewirkt.

Die Erfindung hat gegenüber dem Stand der Technik den Vorteil, dass sie einfach und sicher in der Anwendung ist. Insbesondere führt der Einsatz der erfindungsgemäßen Protektionsvorrichtung sowohl bei der Platzierung als auch bei der Entfernung zu keiner zusätzlichen Gefährdung des Patienten durch die Freisetzung von thrombembolischem Material.

Dadurch, dass die retrograde Platzierung weiterer Instrumente nicht durch eine spezielle Öffnung der Protektionsvorrichtung, sondern zwischen der Protektionsvorrichtung und der Gefäßwand erfolgt, kann die erfindungsgemäße Protektionsvorrichtung bei einer Vielzahl von entsprechenden Interventionen genutzt werden, ohne sich bei der Auswahl der weiteren Instrumente auf solche mit einem bestimmten Kaliber beschränken zu müssen.Die Erfindung sowie das technische Umfeld werden nachfolgend anhand der Figuren näher erläutert. Es ist darauf hinzuweisen, dass die Figuren eine besonders bevorzugte Ausführungsvariante der Erfindung zeigen. Die Erfindung ist jedoch nicht auf die gezeigte Ausführungsvariante beschränkt. Insbesondere umfasst die Erfindung, soweit es technisch sinnvoll ist, beliebige Kombinationen der technischen Merkmale, die in den Ansprüchen aufgeführt oder in der Beschreibung als erfindungsrelevant beschrieben sind.

Es zeigen:
- Fig. 1: eine bevorzugte Ausführungsform der erfindungsgemäßen Protektionsvorrichtung
- Fig. 2: die Ausführungsform gemäß Fig. 2 im implantierten Zustand

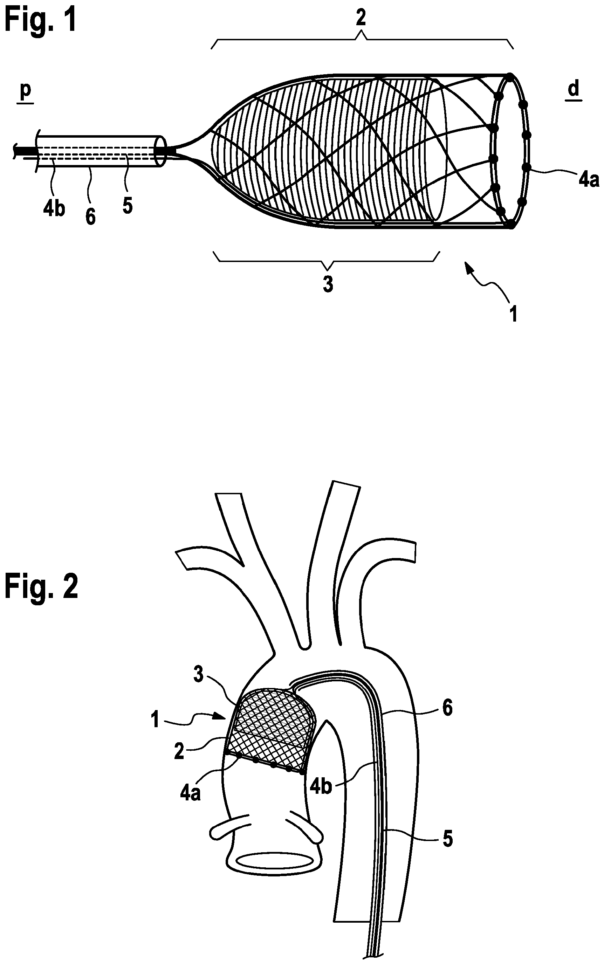

Figur 1 zeigt eine bevorzugte Ausführungsform der erfindungsgemäßen Protektionsvorrichtung 1 mit einem Protektionselement umfassend ein Stützelement 2 und ein an dem Stützelement 2 angeordnetes Filterelement 3. Das Filterelement 3 ist vorzugsweise innerhalb des Stützelements 2 angeordnet und legt sich an die Innenseite des Stützelements 2 an. Stützelement 2 und Filterelement 3 können auf übliche Weisen miteinander verbunden sein.

Das Stützelement 2 kann reversibel einen komprimierten und einen expandierten Zustand einnehmen. Entsprechend kann auch das Protektionselement insgesamt einen komprimierten und einen expandierten Zustand einnehmen.

Das Protektionselement ist im dargestellten expandierten Zustand nach distal geöffnet und nach proximal geschlossen. Am proximalen Ende des Stützelements 2 ist ein Zuführelement 5 vorgesehen, mit dem das Protektionselement durch einen Zuführkatheter 6 geführt werden kann. Innerhalb des Zuführkatheters 6 nimmt das Protektionselement seinen komprimierten und außerhalb des Zuführkatheters 6 seinen expandierten Zustand ein.

Das Stützelement 2 ist selbstexpandierbar vorgesehen und kann beispielsweise Formgedächtnislegierungen oder sonstigen Legierungen umfassen, aus denen durch Schneiden oder Flechten selbstexpandierende Elemente hergestellt werden können.

Das Filterelement 3 kann beispielsweise Gewebe, Membranen oder Fleece mit einer bestimmten Porengröße umfassen, wobei die Porengröße und somit die letztendliche Materialauswahl den jeweiligen Anforderungen anzupassen ist. In der Regel liegt die Porengröße zwischen 50 und 500 µm, vorzugsweise zwischen 60 und 250 µm und insbesondere 100 und 150 µm.

Das Filterelement 3 kleidet das Stützelement 2 zumindest in dessen proximalem Bereich aus, es kann das Stützelement 2 jedoch auch vollständig auskleiden.

Die Protektionsvorrichtung 1 kann wie dargestellt eine Verschlussvorrichtung 4 umfassen, die vorgesehen ist, einen Verschluss der distalen Öffnung des Protektionselements beziehungsweise des Stützelements 2 bereits vor dem Zurückziehen des Protektionselements in den Zuführkatheter 6 zu ermöglichen. Dies ist vorteilhaft, damit bereits während des Zurückziehens kein in dem Filterelement 3 eingefangenes Material aus dem Protektionselement austreten und gegebenenfalls zu Embolien führen kann.

In der dargestellten bevorzugten Ausführungsform der Verschlussvorrichtung 4 umfasst die Verschlussvorrichtung 4 Halteelemente 4a und zumindest ein Zugelement 4b. Die Halteelemente 4a sind am distalen Ende des Stützelements 2 vorgesehen. Sie können Teil des Stützelements 2 sein oder an das Stützelement 2 angefügt sein. Die Halteelemente 4a sind zur Aufnahme des Zugelements 4b vorgesehen. Das Zugelement 4b ist bandförmig mit zwei Enden vorgesehen. Vorzugsweise sind die Haltelemente 4a als Öffnungen oder Ösen vorgesehen, durch die das Zugelement 4b geführt wird.

Zumindest ein Ende des Zugelements 4b wird so nach proximal durch den Zuführkatheter 6 geführt, dass dieses eine Ende des Zugelements 4b durch den Anwender zum Verschluss des Protektionselements beziehungsweise des Stützelements 2 genutzt werden kann. Das andere Ende des Zugelements 4b kann ebenfalls nach proximal durch den Zuführkatheter 6 geführt werden, sodass auch dieses andere Ende des Zugelements 4b durch den Anwender zum Verschluss des Stützelements 2 genutzt werden kann. Das Zugelement 4b bildet in diesem Fall insgesamt eine Schlinge aus. Alternativ kann das andere Ende des Zugelements 4b auch an einer Stelle der Protektionsvorrichtung 1, vor allem an einer Stelle des Stützelements 2 oder an einem der Halteelemente 4a, festgelegt sein.

Figur 2 zeigt beispielhaft die in der Aorta ascendens platzierte erfindungsgemäße Protektionsvorrichtung 1. Das Protektionselement mit Stützelement 2 und Filterelement 3 liegt formschlüssig in der Aorta ascendens proximal des Abgangs der Koronararterien und distal des Abgangs der hirnversorgenden Gefäße. Hierdurch ist gewährleistet, dass während der Intervention kein abgelöstes Material in die zerebralen Abgänge oder in den systemischen Blutkreislauf gelangen kann.

Das mit der Protektionsvorrichtung 1 zu fangende thrombembolische Material kann nach der Intervention durch den distalen Verschluss 4 des Stützelements 2 sicher geborgen und aus dem Körper des Patienten entfernt werden. Es ergeben sich durch die Lage des Devices kaum Gefahren der sekundären Plaquemobilisation aus dem Aortenbogen und der Aorta descendens im Rahmen der Entfernung desselben.

### Bezuaszeichenliste

- 1: Protektionsvorrichtung
- 2: Stützelement
- 3: Filterelement
- 4: Verschlussvorrichtung (4a: Halteelement; 4b: Zugelement)
- 5: Zuführelement
- 6: Zuführkatheter/Schleuse

## Patentansprüche

1. Endovaskuläre Protektionsvorrichtung mit
- einem Protektionselement, umfassend ein selbstexpandierbares Stützelement (2) und ein an dem Stützelement (2) angeordnetes Filterelement (3), und
- einem proximal an dem Stützelement (2) angeordneten Zuführelement (5),
wobei das Protektionselement reversibel von einem expandierten in einen komprimierten Zustand überführbar ist, **dadurch gekennzeichnet, dass** das Filterelement (3) zumindest im expandierten Zustand an seinem distalen Ende geöffnet und an seinem proximalen Ende geschlossen ist.

2. Endovaskuläre Protektionsvorrichtung nach Anspruch 1, **dadurch gekennzeichnet, dass** sich das Stützelement (2) nach proximal und/oder distal verjüngt.

3. Endovaskuläre Protektionsvorrichtung nach Anspruch 1 oder 2, **dadurch gekennzeichnet, dass** das Filterelement (3) eine Porengröße zwischen 50 und 500 µm, vorzugsweise zwischen 60 und 250 µm und insbesondere zwischen 100 und 150 µm aufweist.

4. Endovaskuläre Protektionsvorrichtung nach einem der vorherigen Ansprüche, **dadurch gekennzeichnet, dass** das Stützelement (2) aus einem Formgedächtnismaterial gefertigt ist.

5. Endovaskuläre Protektionsvorrichtung nach Anspruch 4, **dadurch gekennzeichnet, dass** das Stützelement (2) durch Flechten oder Laserschneiden erhältlich ist.

6. Endovaskuläre Protektionsvorrichtung nach einem der vorherigen Ansprüche, **dadurch gekennzeichnet, dass** die endovaskuläre Protektionsvorrichtung weiterhin eine Verschlussvorrichtung (4, 4a, 4b) zum distalen Verschluss des Filter- und/oder Stützelements (2, 3) umfasst.

7. Endovaskuläre Protektionsvorrichtung nach einem der vorherigen Ansprüche, **dadurch gekennzeichnet, dass** das Stützelement (2) selbstexpandierend vorgesehen ist.

8. Endovaskuläre Protektionsvorrichtung nach einem der vorherigen Ansprüche, **dadurch gekennzeichnet, dass** das Stützelement (2) aus einem Formgedächtnismaterial vorgesehen ist.

9. Endovaskuläre Protektionsvorrichtung nach einem der vorherigen Ansprüche, **dadurch gekennzeichnet, dass** das Filterelement (3) aus einem biokompatiblem Material vorgesehen ist.

10. Endovaskuläre Protektionsvorrichtung nach einem der vorherigen Ansprüche, **dadurch gekennzeichnet, dass** das Filterelement (3) aus PE, PTFE oder ePTFE vorgesehen ist.
